# EUROPEAN PATENT APPLICATION

(11) **EP 3 738 959 A1**
(43) Date of publication of application: **18.11.2020**
(21) Application number: 19174826.8
(22) Date of filing: 16.05.2019
(51) Int. Cl.: C07D 401/06, C07D 213/643

(54) **PREPARATION OF 6-PHENOXY-2-(HALOALKYL)-3-ACYLPYRIDINES AND INTERMEDIATES THEREFOR**

(71) Applicant: Bayer AG, 51373 Leverkusen (DE)
(72) Inventor: PAZENOK, Sergii, 42699 Solingen (DE); DRÖGE, Thomas, 40764 Langenfeld (DE)
(74) Representative: BIP Patents

(57) **Abstract**

The present invention relates to a process for preparing compounds of formula (I) 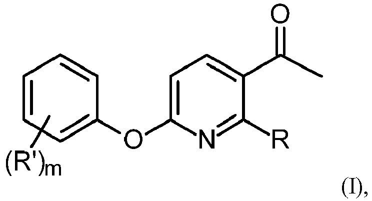 wherein R represents C₁-C₂-haloalkyl, R' represents halogen, CN, nitro, C₁-C₄-alkyl, C₁-C₄-haloalkyl, C₁-C₄-alkoxy, C₁-C₄-haloalkoxy, C₁-C₄-alkylcarbonyl, hydroxy-substituted C₁-C₄-alkyl or pentafluoro-λ⁶-sulfanyl and m is an integer and is 0, 1, 2, 3, 4 or 5, by reacting a compound of formula (II) 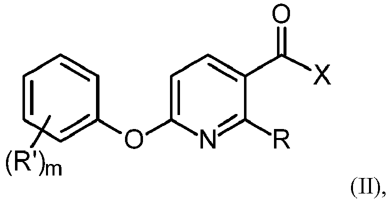 wherein R, R' and m are defined as in formula (I) and X represents chlorine or bromine, in a first step A1) with a malonate of formula (III) 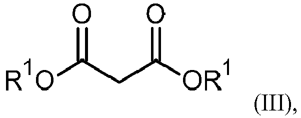 wherein R¹ represents C₁-C₆-alkyl, C₂-C₆-alkenyl, C₂-C₆-alkynyl, C₃-C₈-cycloalkyl or benzyl, in presence of a base and a magnesium compound, and decarboxylating the resulting compound in a second step A2). Optionally the resulting compound of formula (I) is further reacted to a triazole derivative of formula (VI)

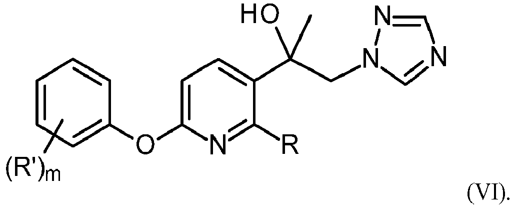

It further relates to the compounds of formula (II), as well as compounds of formulae (IVa) and (IVb).

## Description

The present invention relates to a process for preparing 6-phenoxy-2-(haloalkyl)-3-acylpyridines, and optionally further reacting such pyridines to triazole derivatives showing fungicidal activity. It further relates to key intermediates of said process.

6-phenoxy-2-(haloalkyl)-3-acylpyridines are known to be valuable intermediates in the synthesis of compounds useful in the field of crop protection. WO 2017/029179 A1 discloses fungicidal triazole compounds and several routes to synthesize those. The routes referred to in WO 2017/029179 A1 as processes A, B, D, and E, respectively, comprise synthesis of 6-phenoxy-2-(haloalkyl)-3-acylpyridines which can be converted to the desired triazole derivatives. Said processes A, B, and E require conversion of suitable halogenated phenoxypyridine derivatives into Grignard compounds and reacting such Grignard compounds with acyl chlorides to the desired 6-phenoxy-2-(haloalkyl)-3-acylpyridines. According to said process D a dihalogenated pyridine is reacted with carbon dioxide or carbon monoxide. In the further procedure process D also uses Grignard compounds to arrive at the desired products. Although such procedures provide access to the target phenoxypyridines they have certain drawbacks, in particular necessity to use an organometallic reagent. In the not prepublished European patent application 18211222.7 an improved process avoiding handling of organometallic reagents is disclosed.

Object of the invention is provision of a further process for the synthesis of 6-phenoxy-2-(haloalkyl)-3-acylpyridines that avoids the drawbacks of the known methods and provides the desired compounds in excellent yield.

Surprisingly, it has been found that 6-phenoxy-2-(haloalkyl)-3-acylpyridines can be synthesized in high yield starting from certain 6-phenoxynicotinic acid halides by reacting those with malonic ester, followed by decarboxylation. This process avoids the need of employing organometallic reagents and the drawbacks associated therewith.

Reaction of certain nicotinic acid halogenides with malonic ester and decarboxylation of the resulting compounds is known from Tetrahedron Vol. 48, No. 42, pp. 9233-9236, 1992 and EP 0 341 478 A2. However, said references do not disclose synthesis of any 2-(haloalkyl)-3-acylpyridines, nor deal with the production of fungicides or intermediates thereof.

Accordingly, subject of this invention is a process for preparing a compound of formula (I) 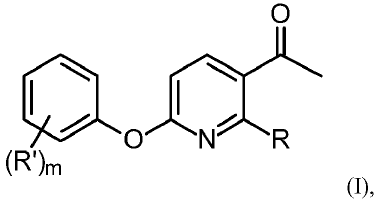 wherein
- R: represents C₁-C₂-haloalkyl;
- R': represents halogen, CN, nitro, C₁-C₄-alkyl, C₁-C₄-haloalkyl, C₁-C₄-alkoxy, C₁-C₄-haloalkoxy, C₁-C₄-alkylcarbonyl, hydroxy-substituted C₁-C₄-alkyl or pentafluoro-λ⁶-sulfanyl; and
- m: is an integer and is 0, 1, 2, 3, 4 or 5;
characterized in that a compound of formula (II) 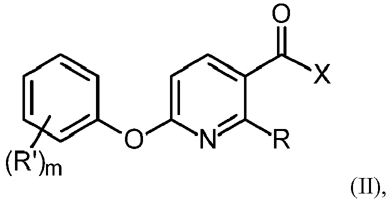 wherein
- R, R': and m are defined as in formula (I); and
- X: represents chlorine or bromine;
is reacted in a first step A1) with a malonate of formula (III) 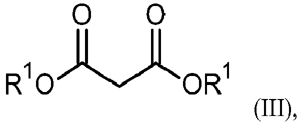 wherein
R¹ represents C₁-C₆-alkyl, C₂-C₆-alkenyl, C₂-C₆-alkynyl, C₃-C₈-cycloalkyl or benzyl;
in presence of a base and a magnesium compound; and
in a second step A2) the resulting compound is decarboxylated.

Formula (I) provides a general definition of the compounds obtainable by the process according to the invention. Preferred radical definitions for formula (I) shown above and below are given below. These definitions apply to compounds of formula (I) and likewise to all educts, intermediates and products bearing the respective radical(s).
- R: preferably represents C₁-haloalkyl.
- R: more preferably represents CF₃, CF₂Cl, CHF₂ or CH₂F.
- R: most preferably represents CF₃.
- R': preferably represents halogen, CN, nitro, C₁-C₄-alkyl, C₁-C₄-haloalkyl, C₁-C₄-alkoxy, C₁-C₄-haloalkoxy or pentafluoro-λ⁶-sulfanyl.
- R': more preferably represents CF₃, OCF₃, Br, Cl or pentafluoro-λ⁶-sulfanyl.
- R': more preferably represents CF₃, OCF₃, Br, Cl or pentafluoro-λ⁶-sulfanyl, wherein the CF₃, OCF₃, Br, Cl or pentafluoro-λ⁶-sulfanyl is attached to the phenyl moiety in the 4-position.
- R': most preferably represents Br or Cl, wherein the Br or Cl is preferably attached to the phenyl moiety in the 4-position.
- m: preferably is 1, 2 or 3.
- m: more preferably is 1 or 2.
- m: most preferably is 1.

Formula (II) and (III) provide a general definition of the educts used in step A1) of the process according to the invention.

Regarding R, R' and m the definitions given with regard to formula (I) apply mutatis mutandis.
- X: most preferably represents chlorine.
- R¹: preferably represents C₁-C₄-alkyl, C₂-C₆-alkenyl, C₂-C₆-alkynyl, cyclopropyl or benzyl.
- R¹: more preferably represents C₁-C₄-alkyl, C₂-C₆-alkenyl, C₂-C₆-alkynyl or cyclopropyl.
- R¹: more preferably represents C₁-C₄-alkyl or cyclopropyl.
- R¹: more preferably represents methyl, ethyl, n-propyl, isopropyl, n-butyl, isobutyl, tert.-butyl or cyclopropyl.
- R¹: more preferably represents methyl, ethyl, n-propyl, isopropyl, n-butyl, isobutyl or tert.-butyl.
- R¹: more preferably represents methyl or ethyl.
- R¹: most preferably represents methyl.

The compounds resulting from step A1) of the process according to the invention can be depicted by formulae (IVa) and/or (IVb)

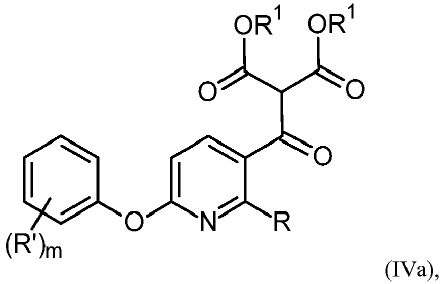

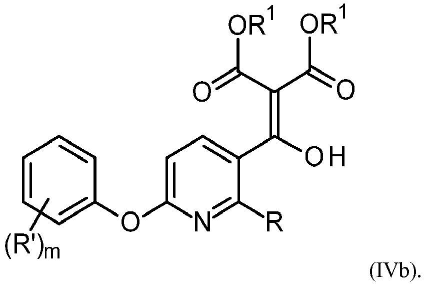

Regarding R, R' and m the definitions given with regard to formula (I) apply mutatis mutandis. Regarding R¹ the definitions given with regard to formula (III) apply mutatis mutandis.

Formulae (IVa) and (IVb) represent tautomeric forms of compounds resulting from step A1) of the process according to the invention. Those compounds can be present as tautomer of formula (IVa), tautomer of formula (IVb) and as mixtures of both isomeric forms in any ratio. They can also be present in any other isomeric, in particular tautomeric, form and as mixture of tautomers of formulae (IVa) and/or (IVb) and any further isomer thereof.

The radical definitions and explanations given above in general terms or stated within preferred ranges can also be combined with one another as desired, i.e. including between the particular ranges and preferred ranges. They apply both to the end products and correspondingly to educts and intermediates. In addition, individual definitions may not apply.

Preference is given to those cases in which each of the radicals have the abovementioned preferred definitions.

Particular preference is given to those cases in which each of the radicals have the abovementioned more and/or most preferred definitions.

Hence, particular preferred is a process for preparing a compound of formula (Ia) 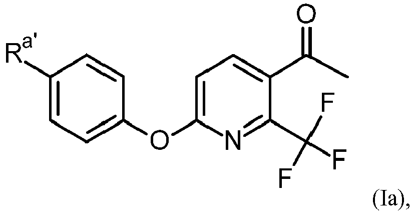 wherein
R^{a}' represents Br or Cl;
characterized in that a compound of formula (IIa) 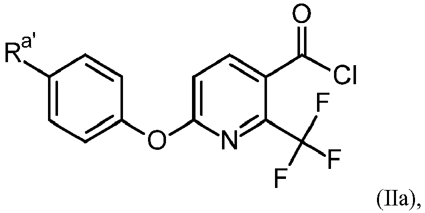 wherein
R^{a}' is defined as in formula (Ia);
is reacted in a first step A1) with the malonate of formula (IIIa) 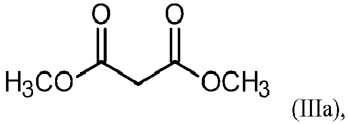 in presence of a base and a magnesium compound; and in a second step A2) the resulting compound is decarboxylated.

In the definitions of the symbols given in the above and below formulae, collective terms were used which are generally representative of the following substituents:
The definition C₁-C₆-alkyl comprises the largest range defined here for an alkyl radical. Specifically, this definition comprises the meanings methyl, ethyl, n-, isopropyl, n-, iso-, sec-, tert-butyl, and also in each case all isomeric pentyls and hexyls, such as methyl, ethyl, propyl, 1-methylethyl, butyl, 1-methylpropyl, 2-methylpropyl, 1,1-dimethylethyl, n-pentyl, 1-methylbutyl, 2-methylbutyl, 3-methylbutyl, 1,2-dimethylpropyl, 1,1-dimethylpropyl, 2,2-dimethylpropyl, 1-ethylpropyl, n-hexyl, 1-methylpentyl, 2-methylpentyl, 3-methylpentyl, 4-methylpentyl, 1,2-dimethylbutyl, 1,3-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl, 2-ethylbutyl, 1-ethyl-3-methylpropyl, in particular propyl, 1-methylethyl, butyl, 1-methylbutyl, 2-methylbutyl, 3-methylbutyl, 1,1-dimethylethyl, 1,2-dimethylbutyl, 1,3-dimethylbutyl, n-pentyl, 1-methylbutyl, 1-ethylpropyl, hexyl, 3-methylpentyl. A preferred range is C₁-C₄-alkyl, such as methyl, ethyl, n-, isopropyl, n-, iso-, sec-, tert-butyl. The definition C₁-C₂-alkyl comprises methyl and ethyl.

The definition halogen comprises fluorine, chlorine, bromine and iodine. Halogen-substitution is generally indicated by the prefix halo, halogen or halogeno.

Halogen-substituted alkyl - e.g. referred to as halogenalkyl, halogenoalkyl or haloalkyl, e.g. C₁-C₄-haloalkyl or C₁-C₂-haloalkyl - represents, for example, C₁-C₄-alkyl or C₁-C₂-alkyl as defined above substituted by one or more halogen substituents which can be the same or different. Preferably C₁-C₄-haloalkyl represents chloromethyl, dichloromethyl, trichloromethyl, fluoromethyl, difluoromethyl, trifluoromethyl, chlorofluoromethyl, dichlorofluoromethyl, chlorodifluoromethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, 1,1-difluoroethyl, pentafluoroethyl, 1-fluoro-1-methylethyl, 2-fluoro-1,1-dimethylethyl, 2-fluoro-1-fluoromethyl-1-methylethyl, 2-fluoro-1,1-di(fluoromethyl)-ethyl, 1-chlorobutyl. Preferably C₁-C₂-haloalkyl represents chloromethyl, dichloromethyl, trichloromethyl, fluoromethyl, difluoromethyl, trifluoromethyl, chlorofluoromethyl, dichlorofluoromethyl, chlorodifluoromethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, 1,1-difluoroethyl, pentafluoroethyl.

The definition C₂-C₆-alkenyl comprises the largest range defined here for an alkenyl radical. Specifically, this definition comprises the meanings ethenyl, n-, isopropenyl, n-, iso-, sec-, tert-butenyl, and also in each case all isomeric pentenyls, hexenyls, 1-methyl-1-propenyl, 1-ethyl-1-butenyl. Halogen-substituted alkenyl - referred to as C₂-C₆-haloalkenyl - represents, for example, C₂-C₆-alkenyl as defined above substituted by one or more halogen substituents which can be the same or different.

The definition C₂-C₆-alkynyl comprises the largest range defined here for an alkynyl radical. Specifically, this definition comprises the meanings ethynyl, n-, isopropynyl, n-, iso-, sec-, tert-butynyl, and also in each case all isomeric pentynyls, hexynyls. Halogen-substituted alkynyl - referred to as C₂-C₆-haloalkynyl - represents, for example, C₂-C₆-alkynyl as defined above substituted by one or more halogen substituents which can be the same or different.

The definition C₃-C₈-cycloalkyl comprises monocyclic saturated hydrocarbyl groups having 3 to 8 carbon ring members, such as cyclopropyl, cyclobutyl, cyclopentyl, cyclohexyl, cycloheptyl and cyclooctyl.

In step A1) of the process according to the present invention a compound of formula (II) is reacted with a malonate of formula (III) in presence of a base and a magnesium compound.

In step A2) of the process according to the present invention the compound resulting from step A1) is decarboxylated.

Decarboxylation as used throughout this specification is to be understood as replacing each of the two C(O)OR¹ moieties present in the compound resulting from step A1) by hydrogen, thereby forming the respective compound of formula (I).

Preferably, in step A1) the malonate of formula (III) is added in a molar amount to arrive at a molar ratio of compound of formula (II) to malonate of formula (III) of 1 : 0.9 to 1 : 3, preferably 1 : 0.95 to 1 : 2, more preferred 1 : 1 to 1 : 1.5, more preferred 1 : 1 to 1 : 1.2, most preferred 1 : 1 to 1 : 1.1.

Throughout this specification any reference to a molar amount of a compound or a molar ratio of two reactants is based on the total amount of the compound and reactants, respectively. Therefore, in case a mixture of a particular compound or reactant is present, the molar amount and molar ratio is based on the total amount of all members of this mixture. For example, if a mixture of two or more malonates of formula (III) is present in step A1), the molar ratio is to be calculated based on the total amount of all present malonates of formula (III). This applies mutatis mutandis for any reference to an amount by weight or volume and any weight or volume ratios.

Preferably, the base present in step A1) is selected from alkali metal or alkaline earth metal acetates, preferably sodium acetate, potassium acetate and calcium acetate, and basic organic nitrogen compounds, preferably trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, pyridine, 2-methyl-, 3-methyl-, 4-methyl-, 2,4-dimethyl-, 2,6-dimethyl-, 3,4-dimethyl- and 3,5-dimethylpyridine, 5-ethyl-2-methylpyridine, 4-dimethylaminopyridine, N-methylpiperidine, 1,4-diazabicyclo[2.2.2]-octane (DABCO), 1,5-diazabicyclo[4.3.0]-non-5-ene (DBN) and 1,8-diazabicyclo[5.4.0]-undec-7-ene (DBU).

More preferably, the base is selected from basic organic nitrogen compounds, preferably trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, 1,4-diazabicyclo[2.2.2]-octane (DABCO), 1,5-diazabicyclo[4.3.0]-non-5-ene (DBN), 1,8-diazabicyclo[5.4.0]-undec-7-ene (DBU) and mixtures thereof, more preferably from trimethylamine, triethylamine, tripropylamine, tributylamine, ethyldiisopropylamine, and mixtures thereof. Most preferred the base is triethylamine.

Preferably, in step A1) the base is present in a molar amount to arrive at a molar ratio of magnesium compound to base of 1 : 1 to 1 : 5, preferably 1 : 1.5 to 1 : 4, more preferred 1 : 2 to 1 : 3.5.

Preferably, the magnesium compound present in step A1) is selected from MgCl₂, MgBr₂, Mg(OH)₂, Mg(OMe)₂, Mg(OEt)₂, MgO, Mg(NO₃)₂ and mixtures thereof, more preferably from MgCl₂, MgBr₂, MgO and mixtures thereof, more preferably from MgCl₂, MgBr₂ and mixtures thereof. Most preferably, the magnesium compound present in step A1) is MgCl₂.

In one particular embodiment of the process according to the invention the magnesium compound present in step A1) is MgO and the base is selected from alkali metal or alkaline earth metal acetates, preferably sodium acetate, potassium acetate, calcium acetate and mixtures thereof.

Preferably, in step A1) the magnesium compound is present in a molar amount to arrive at a molar ratio of compound of formula (II) to magnesium compound of 1 : 0.2 to 1 : 1.5, preferably 1 : 0.3 to 1 : 1.4, more preferred 1 : 0.35 to 1 : 1, more preferred 1 : 0.4 to 1 : 0.8, most preferred 1 : 0.5 to 1 : 0.75.

Preferably, step A1) is carried out in the presence of an aprotic solvent, preferably selected from acetonitrile, ethylacetate, aromatic hydrocarbons, chlorinated aromatic hydrocarbons and mixtures thereof, more preferably selected from acetonitrile, ethylacetate, alkylbenzenes, chlorobenzenes and mixtures thereof, more preferably selected from acetonitrile, ethylacetate, toluene, xylenes, mesitylene, chlorobenzene and mixtures thereof, most preferred selected from toluene, xylenes, mixtures thereof, and mixtures thereof with acetonitrile or ethylacetate.

Preferably, step A1) is carried out at a temperature of -20°C to 50°C, preferably 0°C to 40°C, more preferred 10°C to 30°C.

After completion of the reaction, the reaction mixture resulting from step A1) is preferably treated with an aqueous acid, preferably an aqueous hydrochloric acid or aqueous sulfuric acid, more preferably aqueous sulfuric acid, most preferably aqueous sulfuric acid having a concentration of 10 to 30 % by weight. Preferably, the aqueous acid is added in such amount to adjust the pH of the resulting aqueous phase to 1-6, preferably 1-3.

If not defined otherwise, any reference to a pH value in this specification refers to the value at room temperature (21°C).

Preferably, resulting organic and aqueous phases are separated and the aqueous phase is extracted with an organic solvent, preferably selected from aromatic hydrocarbons, chlorinated aromatic hydrocarbons and mixtures thereof, more preferably selected from alkylbenzenes, chlorobenzenes and mixtures thereof, more preferably selected from toluene, xylenes, mesitylene, chlorobenzene and mixtures thereof, most preferred from toluene and/or xylenes.

The combined organic phases can be used for the decarboxylation step A2) without any further purification.

If, however, it is desired to isolate the resulting compound, this can be achieved by simply removing any organic solvent and volatiles by evaporation in vacuum. If desired, the resulting compounds may be further purified by known techniques, for example chromatography.

In step A2) the product resulting from step A1) is decarboxylated to arrive at the desired compound of formula (I).

Decarboxylation is preferably performed by heating the product resulting from step A1) to at least 50°C, preferably 50°C to 180°C, more preferred 80°C to 170°C, more preferred 85°C to 160°C, most preferred 90°C to 150°C in the presence of water.

Preferably, water is added in an amount of 1 to 30 mol, preferably 2 to 20 mol, more preferred 2 to 10 mol per mol of compound of formula (IVa) and/or (IVb).

Since at the elevated temperature used in step A2) some of the water may evaporate from the reaction mixture, it is preferred to add the desired amount of water in several, e.g. 2 to 10, portions or continuously during stirring of the heated reaction mixture.

Optionally, step A2) is performed in the presence of an acid, preferably an aqueous hydrochloric acid or aqueous sulfuric acid, more preferably aqueous sulfuric acid, most preferably aqueous sulfuric acid having a concentration of 10 to 30 % by weight.

Optionally, step A2) is carried out in the presence of an organic solvent, preferably selected from dimethylsulfoxide (DMSO), *N,N*-dimethylformamide (DMF), *N,N*-dimethylacetamide (DMA), acetonitrile, aromatic hydrocarbons, chlorinated aromatic hydrocarbons and mixtures thereof, more preferably selected from alkylbenzenes, chlorobenzenes and mixtures thereof, more preferably selected from toluene, xylenes, mesitylene, chlorobenzene and mixtures thereof, most preferred in the presence of toluene and/or xylenes. More preferably, the same kind of solvent that is present in step A1), if any, is also used in step A2).

However, preferably step A2) is carried out in the absence of an organic solvent, i.e. organic solvent present in step A1), if any, is removed from the reaction mixture as outlined above and the decarboxylation is preferably performed in the presence of water and optionally an acid only.

Preferably, steps A1) and A2) are performed under standard pressure (1 atm) or under elevated pressure (> 1 atm), more preferably at least step A2) is performed under elevated pressure, preferably at a pressure of 1.2 to 10 bar, more preferred 1.4 to 8 bar, most preferred 1.5 to 5 bar. Working at elevated pressure allows high rection temperatures, like 110 to 130°C, when the reaction is performed in a mixture of organic solvent and water forming an azeotropic mixture, for example toluene/water or xylene/water.

The compound of formula (I) or solution thereof resulting from step A2) can be directly used in step C) of the process outlined below. It is also possible to isolate the compound of formula (I), preferably by evaporation of the organic solvent, preferably under reduced pressure. The process according to the invention yields the compounds of formula (I) in sufficient purity. However, if desired, the compounds of formula (I) may be further purified by known techniques, for example distillation or chromatography. Preferably, the compounds of formula (I) are purified by distillation before use thereof in step C).

The malonates of formula (III), the bases and magnesium compounds used in step A1) are well-known compounds being commercially available or readily obtainable by established synthesis routes.

Compounds of formula (II) used in step A1) can be synthesized from respective compounds of formula (V) 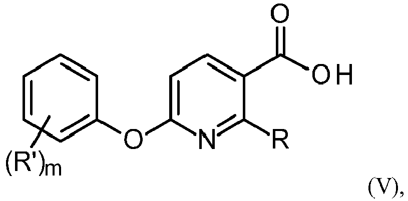 wherein
R, R' and m are defined as in formula (II).

Preferably the compound of formula (II) is prepared by reacting a compound of formula (V) 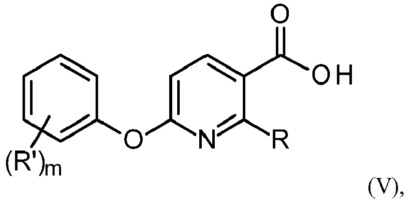 wherein
R, R' and m are defined as in formula (II);
with a dehydroxyhalogenation agent selected from COCl₂, diphosgene, triphosgene, cyanuric chloride, SOCl₂, SO₂Cl₂, PCl₃, PCl₅, POCl₃, PBr₃, SOBr₂ and SO₂Br₂.

In the following this reaction step is referred to as step B).

Preferably, the dehydroxyhalogenation agent used in step B) is selected from POCl₃, COCl₂, diphosgene, triphosgene and SOCl₂, more preferably from POCl₃, COCl₂ and SOCl₂, more preferably the dehydroxyhalogenation agent is POCl₃ or SOCl₂, most preferably the dehydroxyhalogenation agent is SOCl₂.

Step B) can be conducted in presence or absence of a formamide catalyst. If present, the formamide catalyst is preferably selected from *N,N*-Dimethylformamide, *N,N*-Diethylformamide, *N,N-*Diisopropylformamide, *N,N*-Di-n-butylformamide and mixtures thereof, more preferably the formamide catalyst is *N,N*-Di-n-butylformamide. However, preferably, step B) is conducted in absence of a formamide catalyst.

If present, the amount of formamide catalyst in step B) is preferably 0.1 to 10 mole %, more preferably 0.5 to 8 mole %, more preferably 1 to 6 mole %, more preferably 1.5 to 5 mole %, most preferably 2 to 4 mole %, each based on the molar amount of compound of formula (II).

Preferably, step B) is carried out in the presence of an aprotic solvent, preferably selected from nitriles, aromatic hydrocarbons, chlorinated or alkoxylated aromatic hydrocarbons and mixtures thereof, more preferably selected from butyronitrile, alkylbenzenes, alkoxybenzenes, chlorobenzenes and mixtures thereof, more preferably selected from butyronitrile, toluene, xylenes, mesitylene, methoxybenzene, chlorobenzene and mixtures thereof, more preferred in the presence of toluene and/or xylenes, most preferred in the presence of xylenes.

Preferably, step B) is carried out at a temperature of 20°C to 150°C, preferably 50°C to 145°C.

Preferably, the compound of formula (II) and the dehydroxyhalogenation agent are reacted in a molar ratio of 1 : 1.5 to 1 : 30, preferably 1 : 2 to 1 : 20, more preferred 1 : 2.2 to 1 : 19, even more preferred 1 : 2.5 to 1 : 4.5.

Preferably, step B) is performed under standard pressure (1 atm). However, it is also possible to work under elevated or reduced pressure.

The reaction mixture resulting from step B) may be worked-up by procedures generally known in the art. For example, after completion of step B) the reaction mixture can be purified by evaporation of volatile components, in particular excess dehydroxyhalogenation agent, preferably under reduced pressure.

If desired to further purify the compound of formula (II), this is possible by known techniques, for example by distillation or treatment with active charcoal and subsequent filtration.

However, a huge advantage of the process according to the invention is that step A1) does not require the compound of formula (II) to be of particular purity. Therefore, it is neither necessary to work-up the reaction mixture resulting from step B), nor to isolate and further purify the compound of formula (II).

Therefore, preferably the reaction mixture resulting from step B) is directly used in step A1) without isolation or purification of the resulting compound of formula (II).

In other words, preferably step B) and step A1) are conducted without isolation or purification of the reaction product resulting from step B).

More preferred, after completion of step B) the reaction mixture is quenched with water at a temperature of 0°C to 30°C, preferably 5°C to 25°C, the resulting phases are separated and the organic phase is used in step A1).

Compounds of formula (V) are accessible by established synthesis routes, for example process D disclosed in WO 2017/029179 A1.

As outlined above, compounds of formula (I) are valuable intermediates in the synthesis of compounds useful in the field of crop protection, in particular the triazole compounds disclosed in WO 2017/029179 A1.

Therefore, the present invention refers furthermore to a process, wherein a compound of formula (I) is synthesized as outlined above and is further reacted to a triazole derivative of formula (VI) 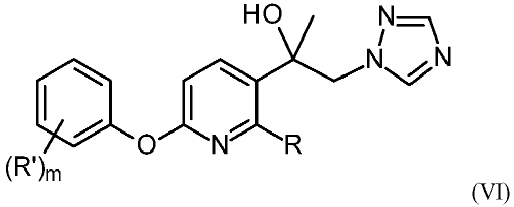 wherein
R, R' and m are defined as in formula (I);
wherein the reaction of the compound of formula (I) to the triazole derivative of formula (VI) comprises the following steps :
step C):
   reacting the compound of formula (I) with a trimethylsulfoxonium halide, a trimethylsulfonium halide, trimethylsulfoxonium methylsulfate or trimethylsulfonium methylsulfate to an epoxide of formula (VII) 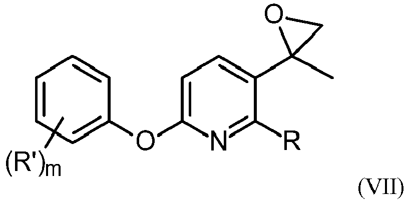 wherein
   R, R' and m are defined as in formula (VI); and
step D):
   reacting the compound of formula (VII) with 1H-1,2,4-triazole in the presence of a base to the triazole derivative of formula (VI).

Regarding R, R' and m the definitions given with regard to formula (I) apply mutatis mutandis.

In step C) compounds of formula (I) are converted into epoxides of formula (VII) by reaction with a trimethylsulfoxonium halide, a trimethylsulfonium halide, trimethylsulfoxonium methylsulfate or trimethylsulfonium methylsulfate, preferably trimethylsulfoxonium chloride, trimethylsulfonium chloride, trimethylsulfoxonium methylsulfate or trimethylsulfonium methylsulfate and a base, such as sodium hydroxide or potassium hydroxide. It is possible to prepare the trimethylsulfoxonium halide, trimethylsulfonium halide, trimethylsulfoxonium methylsulfate or trimethylsulfonium methylsulfate separately before using it in step C). However, it is preferred to prepare said reagents *in situ,* e.g. trimethylsulfonium methylsulfate from a mixture of dimethylsulfide and dimethylsulfate, preferably in the presence of water.

The trimethylsulfoxonium halide, trimethylsulfonium halide, trimethylsulfoxonium methylsulfate or trimethylsulfonium methylsulfate is preferably used in an amount of 1.1 to 2.5, in particular 1.2 to 2, more preferred 1.3 to 1.6 mole equivalents per 1 mole of compound of formula (I).

Preferably, trimethylsulfonium methylsulfate is used. Particularly preferred an aqueous solution of trimethylsulfonium methylsulfate is used, preferably an aqueous solution containing 38 to 40 wt%, preferably 38 to 39.5 wt%, more preferred 38 to 39.0 wt% of trimethylsulfonium cation. Preferably, said trimethylsulfonium methylsulfate is prepared *in situ,* preferably by mixing dimethylsulfide and water and adding dimethylsulfate to said mixture. Preferably, dimethylsulfide is used in excess, more preferred in an amount resulting in a molar ratio of dimethylsulfate to dimethylsulfide of 1 : 1.5 to 1 : 100, preferably 1 : 2 to 1 : 100, more preferred 1 : 3 to 1 : 90, more preferred 1 : 3.5 to 1 : 80, most preferred 1 : 3.5 to 1 : 5.

Preferably, step C) is carried out at a temperature of -30°C to 50°C, preferably -10°C to 40°C, particularly preferred 20°C to 40°C.

Step C) is preferably conducted in the presence of water, dimethylsulfide, acetonitrile, xylenes, toluene or a mixture thereof, more preferably in the presence of water, dimethylsulfide or a mixture thereof.

It is preferably carried out in the presence of a base. Preferably the base is selected from Na₂CO₃, K₂CO₃, Cs₂CO₃, NaOH, KOH, KOtBu, NaH and mixtures thereof, more preferably the base is KOH.

The reaction mixture resulting from step C) can be worked-up by procedures generally known in the art. Preferably, after completion of the reaction, the reaction mixture is quenched by addition of water. Preferably, resulting organic and aqueous phases are separated, the aqueous phase is extracted with an organic solvent, preferably *tert-butyl* methyl ether. The resulting solution of the compound of formula (VII) or the crude product obtained by evaporation of the organic solvent and other volatile components can be directly used in step D) of the process according to the invention. However, if desired, the compounds of formula (VII) may be further purified by known techniques, for example recrystallization or chromatography.

Preferably, in step D) the epoxide of formula (VII) and 1H-1,2,4-triazole are reacted in a molar ratio of 1 : 1 to 1 : 10, preferably 1 : 1 to 1 : 5, more preferred 1 : 1 to 1 : 3, even more preferred 1 : 1 to 1 : 1.8, most preferred 1 : 1.1 to 1 : 1.2.

Step D) is carried out in the presence of a base. These preferably include alkali metal or alkaline earth metal acetates, amides, carbonates, hydrogencarbonates, hydrides, hydroxides or alkoxides, for example sodium acetate, potassium acetate or calcium acetate, lithium amide, sodium amide, potassium amide or calcium amide, sodium carbonate, potassium carbonate or calcium carbonate, sodium hydrogencarbonate, potassium hydrogencarbonate or calcium hydrogencarbonate, lithium hydride, sodium hydride, potassium hydride or calcium hydride, lithium hydroxide, sodium hydroxide, potassium hydroxide or calcium hydroxide, n-butyllithium, sec-butyllithium, tert-butyllithium, lithium diisopropylamide, lithium bis(trimethylsilyl)amide, sodium methoxide, ethoxide, n- or i-propoxide, n-, i-, s- or t-butoxide or potassium methoxide, ethoxide, n- or i-propoxide, n-, i-, s- or t-butoxide; and also basic organic nitrogen compounds, for example trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, pyridine, 2-methyl-, 3-methyl-, 4-methyl-, 2,4-dimethyl-, 2,6-dimethyl-, 3,4-dimethyl- and 3,5-dimethylpyridine, 5-ethyl-2-methylpyridine, 4-dimethylaminopyridine, N-methylpiperidine, 1,4-diazabicyclo[2.2.2]-octane (DABCO), 1,5-diazabicyclo[4.3.0]-non-5-ene (DBN) or 1,8-diazabicyclo[5.4.0]-undec-7-ene (DBU).

Preferably the base is selected from Na₂CO₃, K₂CO₃, Cs₂CO₃, NaOH, KOH, KOtBu, NaH and mixtures thereof, more preferably from KOH, K₂CO₃, Cs₂CO₃ and mixtures thereof.

In a particular embodiment of this step, a sodium or potassium salt of 1H-1,2,4-triazole is used as base. Said sodium or potassium salt can be prepared by reacting 1H-1,2,4-triazole and a sodium or potassium base, preferably selected from NaOH, NaH and Na-alcoholates or KOH and K-alcoholates, respectively.

Preferably, the base is used in at most stoichiometric amount with respect to the epoxide of formula (VII). More preferred, the epoxide of formula (VII) and the base are reacted in a molar ratio of 1 : 1 to 1 : 0.05, preferably 1 : 0.8 to 1 : 0.05, more preferred 1 : 0.5 to 1 : 0.05, even more preferred 1 : 0.3 to 1 : 0.05.

Preferably, step D) is carried out in the presence of an organic solvent, preferably selected from tetrahydrofuran, methyltetrahydrofuran, in particular 2-methyltetrahydrofuran, diethylether, cyclopropyl methyl ether, tert-butyl methyl ether, toluene, C₁-C₆-alcohols, preferably n-butanol, n-propanol, isopropanol, ethanol and methanol, C₁-C₄-alkoxy-C₁-C₆-alcohols, preferably 1-methoxy-propan-2-ol, N-methylpyrrolidinone (NMP), dimethylformamide (DMF), dimethylacetamide (DMAc), dimethylsulphoxide (DMSO), dimethylpropyleneurea (DMPU), dimethylethyleneurea (DMI), tetramethylurea (TMU), N-methylimidazole (NMI), acetonitrile, propionitrile, butyronitrile and mixtures thereof, more preferably in the presence of n-butanol.

Preferably, step D) is carried out at a temperature of 20°C to 150°C, preferably 120°C to 150°C, particularly preferred 100°C to 120°C.

The reaction mixture resulting from step D) can be worked-up by procedures generally known in the art. Preferably, after completion of the reaction, all volatile compounds are evaporated under reduced pressure and the residue is re-dissolved in a suitable organic solvent like dichloromethane or ethyl acetate. Water is added and, if needed, the pH (room temperature) is adjusted to about 6 by introduction of a strong acid like concentrated aqueous hydrochloric acid. The aqueous phase is preferably extracted with a suitable organic solvent like dichloromethane or ethyl acetate. Optionally, the combined organic phases are dried, preferably over magnesium sulfate or sodium sulfate. In case the organic solvent used is dichloromethane drying of the organic phases is not necessary. Preferably, the organic solvent is removed by evaporation and the resulting crude product further purified by known techniques, for example recrystallization or chromatography.

The reaction time of each of the steps of the process according to the invention varies depending on the scale of the reaction and the reaction temperature, but is generally between a few, e.g. 5, minutes and 48 hours.

If not defined otherwise, the process steps according to the invention are generally performed under standard pressure (1 atm). However, it is also possible to work under elevated or reduced pressure.

If not defined otherwise, the amount of any solvent present in any process step according to the invention can vary within wide limits, e.g. from 1 to 99 % by weight, based on the total weight of the respective reaction mixture. Preferrably, the amount of any solvent present in any process step according to the invention is from 1 to 95 % by weight, more preferably from 2 to 90 % by weight, more preferably from 3 to 85 % by weight, more preferably from 4 to 85 % by weight, more preferably from 5 to 80 % by weight, more preferably from 10 to 80 % by weight, more preferably from 15 to 70 % by weight, more preferably from 20 to 70 % by weight, each based on the total weight of the respective reaction mixture.

The invention further relates to compounds of formula (IVa) and (IVb) 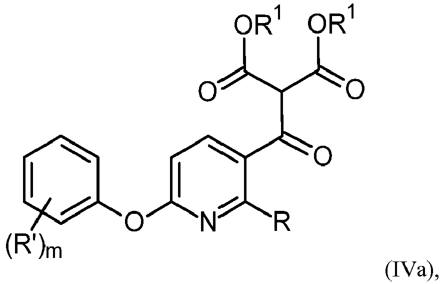 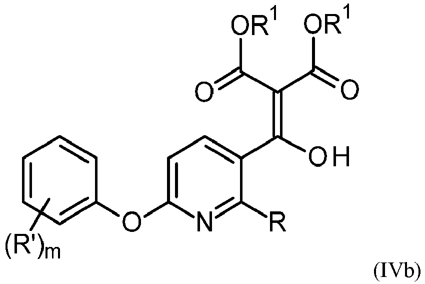 wherein
- R: represents C₁-C₂-haloalkyl;
- R': represents halogen, CN, nitro, C₁-C₄-alkyl, C₁-C₄-haloalkyl, C₁-C₄-alkoxy, C₁-C₄-haloalkoxy, C₁-C₄-alkylcarbonyl, hydroxy-substituted C₁-C₄-alkyl or pentafluoro-λ⁶-sulfanyl;
- m: is an integer and is 0, 1, 2, 3, 4 or 5; and
- R¹: represents C₁-C₆-alkyl, C₂-C₆-alkenyl, C₂-C₆-alkynyl, C₃-C₈-cycloalkyl or benzyl.

As outlined above, formulae (IVa) and (IVb) do not only encompass the depicted tautomeric forms, but also mixtures thereof, as well as any mixtures with any other isomeric, in particular tautomeric form, such as, for example, tautomers of formula (IVc) 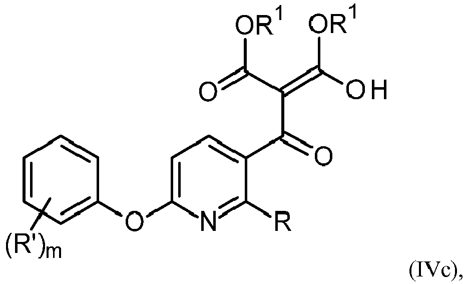 wherein R, R', m and R¹ are defined as in formulae (IVa) and (IVb).

Preferred are compounds of formula (IVa) and (IVb), wherein R represents C₁-haloalkyl, preferably CF₃, CF₂Cl, CHF₂ or CH₂F, more preferably CF₃.

Preferred are also compounds of formula (IVa) and (IVb), wherein R' represents halogen, CN, nitro, C₁-C₄-alkyl, C₁-C₄-haloalkyl, C₁-C₄-alkoxy, C₁-C₄-haloalkoxy or pentafluoro-λ⁶-sulfanyl, preferably represents CF₃, OCF₃, Br, Cl or pentafluoro-λ⁶-sulfanyl, more preferably represents CF₃, OCF₃, Br, Cl or pentafluoro-λ⁶-sulfanyl in the 4-position of the phenyl moiety, most preferably represents Br or Cl, preferably in the 4-position of the phenyl moiety.

Preferred are also compounds of formula (IVa) and (IVb), wherein m is 1, 2 or 3, preferably is 1 or 2, most preferably is 1.

Preferred are also compounds of formula (IVa) and (IVb), wherein R¹ represents C₁-C₄-alkyl, C₂-C₆-alkenyl, C₂-C₆-alkynyl, cyclopropyl or benzyl, preferably C₁-C₄-alkyl, C₂-C₆-alkenyl, C₂-C₆-alkynyl or cyclopropyl, more preferably C₁-C₄-alkyl or cyclopropyl, more preferably methyl, ethyl, n-propyl, isopropyl, n-butyl, isobutyl, tert.-butyl or cyclopropyl, more preferably methyl, ethyl, n-propyl, isopropyl, n-butyl, isobutyl or tert.-butyl, more preferably methyl or ethyl, most preferably methyl.

Preference is given to those compounds of formula (IVa) and (IVb) in which each of the radicals have the abovementioned preferred definitions.

Particular preference is given to those compounds of formula (IVa) and (IVb) in which each of the radicals have the abovementioned more and/or most preferred definitions.

Hence, particular preferred are the compounds of formula (IVa) and (IVb), wherein
- R: represents CF₃;
- R': represents chlorine or bromine in the 4-position of the phenyl ring;
- m: is 1; and
- R¹: represents CH₃.

Compounds of formula (IVa) and (IVb) are obtainable by step A1) of the process according to the invention as outlined above and are key intermediates in said process.

The invention further relates to compounds of formula (II) 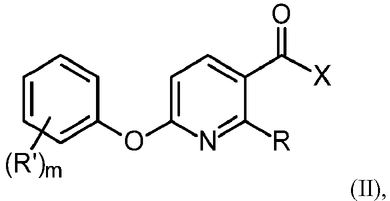 wherein
- R: represents C₁-C₂-haloalkyl;
- R': represents halogen, CN, nitro, C₁-C₄-alkyl, C₁-C₄-haloalkyl, C₁-C₄-alkoxy, C₁-C₄-haloalkoxy, C₁-C₄-alkylcarbonyl, hydroxy-substituted C₁-C₄-alkyl or pentafluoro-λ⁶-sulfanyl;
- m: is an integer and is 0, 1, 2, 3, 4 or 5; and
- X: represents chlorine or bromine.

Preferred are compounds of formula (II), wherein R represents C₁-haloalkyl, preferably CF₃, CF₂Cl, CHF₂ or CH₂F, more preferably CF₃.

Preferred are also compounds of formula (II), wherein R' represents halogen, CN, nitro, C₁-C₄-alkyl, C₁-C₄-haloalkyl, C₁-C₄-alkoxy, C₁-C₄-haloalkoxy or pentafluoro-λ⁶-sulfanyl, preferably represents CF₃, OCF₃, Br, Cl or pentafluoro-λ⁶-sulfanyl, more preferably represents CF₃, OCF₃, Br, Cl or pentafluoro-λ⁶-sulfanyl in the 4-position of the phenyl moiety, most preferably represents Br or Cl, preferably in the 4-position of the phenyl moiety.

Preferred are also compounds of formula (II), wherein m is 1, 2 or 3, preferably is 1 or 2, most preferably is 1.

Preferred are also compounds of formula (II), wherein X represents chlorine.

Preference is given to those compounds of formula (II) in which each of the radicals have the abovementioned preferred definitions.

Particular preference is given to those compounds of formula (II) in which each of the radicals have the abovementioned more and/or most preferred definitions.

Hence, particular preferred are the compounds of formula (II), wherein
- R: represents CF₃;
- R': represents chlorine or bromine in the 4-position of the phenyl ring;
- m: is 1; and
- X: represents chlorine.

Compounds of formula (II) are obtainable by step B) of the process according to the invention as outlined above.

The invention is illustrated by the examples below. However, the invention is not limited to the examples.

### Examples

### Example 1: Step A1) of the process according to the invention

### Preparation of a tautomeric mixture of dimethyl 2-[[6-(4-bromophenoxy)-2-(trifluoromethyl)-3-pyridyl]-hydroxy-methylenejpropanedioate and dimethyl 2-[6-(4-bromophenoxy)-2-(trifluoromethyl)pyridine-3-carbonyl]propanedioate in a molar ratio of 79:21

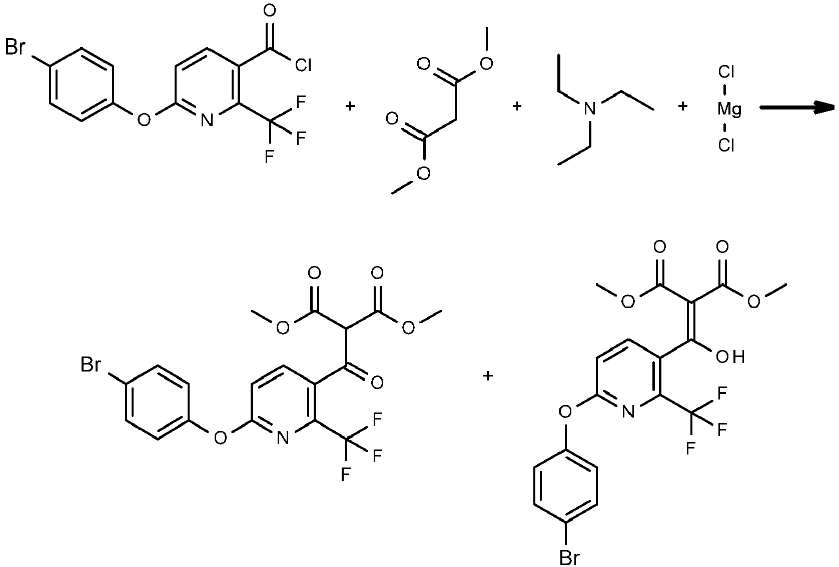

6.65 g (0.07 mol) water free MgCl₂, 13.8 g (0.105 mol) dimethylmalonate and 22 g (0.22 mol) triethylamine in 100 ml xylene were stirred 1 h at room temperature (21°C) to form a white suspension. 38 g (0.1 mol) 6-(4-bromophenoxy)-2-(trifluoromethyl)pyridine-3-carbonyl chloride dissolved in 10 ml of xylene was added to the suspension at 20-22°C within 1-2 h and the mixture was stirred additionally for 1 h. The mixture was acidified with 140 g of 20 % by weight H₂SO₄ and stirred for 20 minutes at room temperature before the phases were separated. The resulting water phase was extracted with 20 ml of xylene and the combined organic extract (110 ml) was used for the decarbolkoxylation step (example 2, step A2)) without purification.

To isolate and characterize the product, a sample of the xylene phase was evaporated in vacuum to give a pale brown oil, being a mixture of two components. Yield: 98 %.

### Analytical characterization:

m/e: 476
Interpretation ofNMR spectra (¹H-NMR, 600.13 MHz, CD₃CN; ¹³C-NMR, 150.95 MHz, CD₃CN):

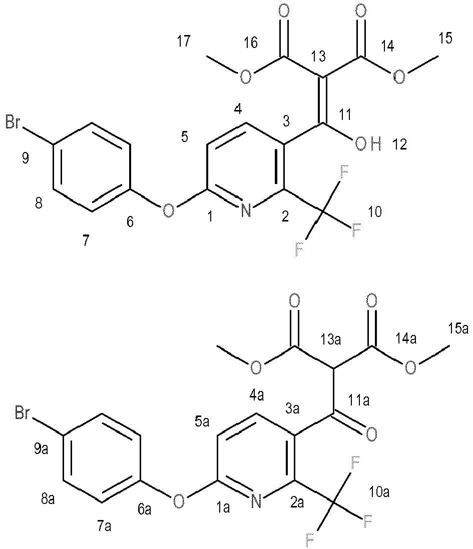

| H/C | δH/ppm | Mult. | rel. No. H | δC/ppm | Mult. | rel. No. C |
|---|---|---|---|---|---|---|
| 1 | - | - | - | 164.0 | S | 1 |
| 1a | - | - | - | 164.3 | S | 1 |
| 2 | - | - | - | 142.7 | S(Q) | 1 |
| 2a | - | - | - | 143.7 | S(Q) | 1 |
| 3 | - | - | - | 125.1 | S(Q) | 1 |
| 3a | - | - | - | 129.3 | S | 1 |
| 4 | 7.87 | D | 1 | 142.9 | D | 1 |
| 4a | 8.14 | D | 1 | 142.1 | D | 1 |
| 5 | 7.25 | D | 1 | 115.7 | D | 1 |
| 5a | 7.26 | D | 1 | 115.5 | D | 1 |
| 6 | - | - | - | 153.3 | S | 1 |
| 6a | - | - | - | 153.1 | S | 1 |
| 7 | 7.16 | M | 2 | 124.5 | D | 2 |
| 7a | 7.16 | M | 2 | 124.5 | D | 1 |
| 8 | 7.60 | M | 2 | 133.7 | D | 2 |
| 8a | 7.60 | M | 2 | 133.8 | D | 2 |
| 9 | - | - | - | 118.8 | S | 1 |
| 9a | - | - | - | 118.9 | S | 1 |
| 10 | - | - | - | 121.9 | S(Q) | 1 |
| 10a | - | - | - | 121.6 | S(Q) | 1 |
| 11 | - | - | - | 175.0 | S | 1 |
| 11a | - | - | - | 191.2 | S | 1 |
| 12 | 13.52 | S, br | 1 | - | - | - |
| 13 | - | - | - | 103.2 | S | 1 |
| 13a | 5.31 | S | 1 | 64.8 | D | 1 |
| 14 | - | - | - | 172.0 | S | 1 |
| 14a | - | - | - | 165.5 | S | 2 |
| 15 | 3.87 | S | 3 | 53.7 | Q | 1 |
| 15a | 3.74 | S | 6 | 54.0 | Q | 2 |
| 16 | - | - | - | 165.3 | S | 1 |
| 17 | 3.45 | S | 3 | 52.6 | Q | 1 |

### Example 2: Step A2) of the process according to the invention

### Preparation of 1-[6-(4-bromophenoxy)-2-(trifluoromethyl)-3-pyridyl]ethanone from the compound mixture resulting from step A1)

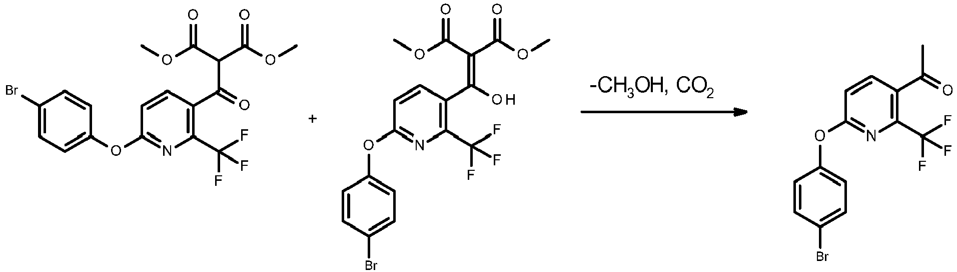

47.6 g of the compound mixture obtained according to example 1 and 250 ml water were heated at 100°C for 20 h. Thereafter the mixture was cooled and the product extracted with ethylacetate. The solvent was removed in vacuum to yield 46.6 g (98 %) of 1-[6-(4-bromophenoxy)-2-(trifluoromethyl)-3-pyridyl]ethanone.

### Example 3: Step B) of the process according to the invention

### Preparation of 6-(4-bromophenoxy)-2-(trifluoromethyl)pyridine-3-carbonyl chloride.

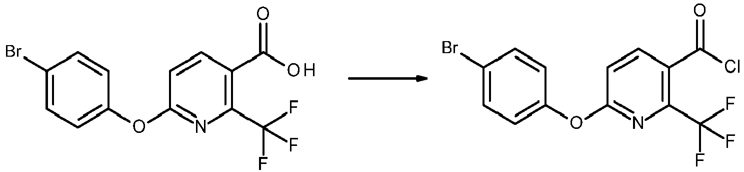

3.62 g (0.01 mol) 6-(4-bromophenoxy)-2-(trifluoromethyl)pyridine-3-carboxylic acid and 1.78 g (0.015 mol) thionyl chloride were heated in 30 ml xylene for 2 h at 100°C. The solvent was removed in vacuo to give 3.8 g of a brown oil (yield = 100 %).
m/e: 375 (analyzed as methylester).

### Example 4:

### Preparation of 6-(4-bromophenoxy)-2-(trifluoromethyl)pyridine-3-carboxylic acid used in example 3

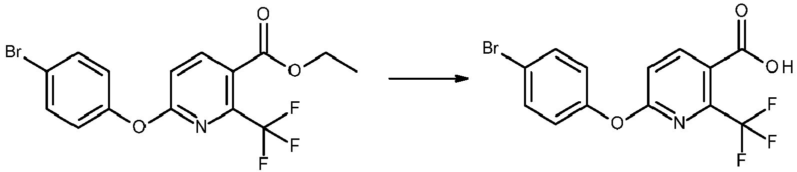

6-(4-bromophenoxy)-2-(trifluoromethyl)pyridine-3-carboxylic acid was prepared from ethyl-6-(4-bromophenoxy)-2-(trifluoromethyl)pyridine-3-carboxylate and aqueous sodium hydroxide (45 w/w%, 2 equivalents) by heating both reagents in THF for 24 h at 60 °C. Thereafter, aqueous HCl (32 w/w%) was added dropwise at room temperature until pH = 2 was reached. The reaction mixture was extracted with methyl *tert-butyl* ether (MTBE) and washed with water. The volatiles were removed to yield an off-white solid. Yield 100 %.
m/e: 360.
¹H NMR (400 MHz; DMSO-d6): δ = 7.23 (m, 2H), 7.43 (d,1H), 7.64 (m,2H), 8.4 (d, 1H) ppm.

### Example 5:

### Preparation of ethyl-6-(4-bromophenoxy)-2-(trifluoromethyl)pyridine-3-carboxylate used in example 4

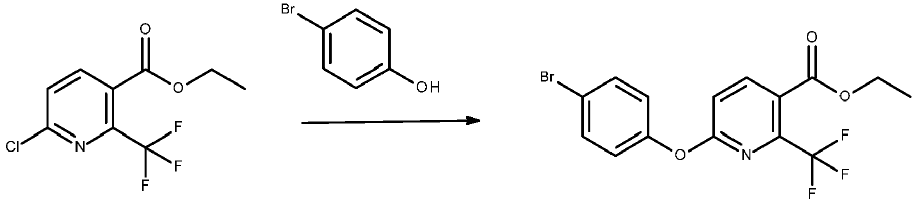

2.5 g (0.015 mol) p-bromophenol and 1.05 g potassium methanolate in 30 ml of toluene were heated to 75°C and the volatiles (CH₃OH) were removed in vacuum at 700 mbar. 2.53 g ethyl 6-chloro-2-(trifluoromethyl)pyridine-3-carboxylate, (0,01 mol), 0.12 g DABCO (diazabicyclo(2,2,2)octane) and 1 ml PEG 400 were added and the reaction mixture was heated at 90°C for 12 h . Afterwards 100 ml of water were added, the phases separated and the resulting water phase extracted with 100 ml methyl *tert-*butyl ether (MTBE). The combined organic fractions were washed with water, dried over Na₂SO₄ and the volatiles were evaporated. The crude product 3.5 g (90 %) was used for the next step without any purification.
m/e: 389.
¹H NMR (400 MHz; DMSO-d6): δ = 1.3 (t, 3H), 4.4 (q, 2H), 7.2 (m, 2H), 7.4 (d,1H), 7.6 (m,2H), 8.3 (d, 1H) ppm.

### Example 6: Steps C) to D) of the process according to the invention

### Preparation of 2-[6-(4-bromophenoxy)-2-(trifluoromethyl)pyridin-3-yl]-1-(1H-1,2,4-triazol-1-yl)propan-2-ol from 1-[6-(4-bromophenoxy)-2-(trifluoromethyl)-3-pyridyl]ethanone

### Step C: 6-(4-bromophenoxy)-3-(2-methyloxiran-2-yl)-2-(trifluoromethyl)pyridine

In a 2 L reactor equipped with an overhead-stirrer, dropping funnel, thermometer and several bleach containing scrubbers 128.6 g dimethylsulfide (2.05 mol, 6.0 equiv) and 6.2 mL water were introduced and heated to 32 °C inner temperature. 65.3 g dimethylsulfate (0.51 mol, 1.5 equiv) was added within 30 minutes. The temperature rose to 39.5 °C and the mixture turned cloudy. The mixture was stirred at 37.5 °C inner temperature for 4 h. The mixture was cooled to room temperature and 124.0 g 1-[6-(4-bromophenoxy)-2-(trifluoromethyl)-3-pyridyl]ethanone (0.34 mol, 1.0 equiv) was added at room temperature. The mixture was stirred 20 minutes before being heated to an inner temperature of 38-40 °C at which 57.5 g KOH pellets (1.03 mol, 3.0 equiv) were added. The mixture was further stirred at 42 °C for 4 h and afterwards cooled to room temperature and kept overnight. HPLC analysis showed full conversion. The mixture was diluted with 400 mL water and 500 mL methyl *tert-butyl* ether (MTBE), filtered through a plug of Celite®, the phases were separated and the organic phase was dried over Na₂SO₄ and evaporated. The crude product was redissolved in 450 mL MTBE and the remaining volatiles were evaporated to obtain 121 g of a redbrown oil of 94.3 a/a-% HPLC purity that crystallizes upon standing (89.3 % yield).
¹H NMR (600 MHz; CD₃CN): δ =8.09, d, (1H); 7.60, d, (2H); 7.22, d, (1H); 7.14, d, (2H); 3.05, d, (1H); 2.90, d, (1H); 1.62 s (3H) ppm.

### Step D: 2- [6-(4-bromophenoxy)-2-(trifluoromethyl)pyridin-3-yl]-1-(1H-1,2,4-triazol-1-yl)propan-2-ol

225 g of 6-(4-bromophenoxy)-3-(2-methyloxiran-2-yl)-2-(trifluoromethyl)pyridine (0.575 mol, 1 equiv) was added to a mixture of 608 mL of n-butanol and 68 mL of PEG400. 60.6 g of 1H-1,2,4-triazole (0.862 mol, 1.5 equiv) and 15.2 g of K₂CO₃ powder (0.23 mol, 0.4 equiv) were added and the mixture was heated to 100 °C for 13 h. The solvent was removed under reduced pressure until a viscous light-brown oil remained. 3 L of ethyl acetate were added together with 1.5 L of water and 18 mL of concentrated aqueous hydrochloric acid. The phases were separated and the organic phase was washed with water. The organic phase was dried over Na₂SO₄ and the solvent was removed under reduced pressure. The remaining oily residue crystallized quickly and was triturated 3 times with cyclohexane. The wet product was filtered and dried, yielding 240 g of beige crystals of 90 a/a-% HPLC purity (84.7% yield). The crystals were dissolved in 760 mL of 2-propanol at 82 °C. 1170 mL of water were added and after stirring at 80 °C for 5 minutes, the mixture was let cool down to room temperature overnight. The suspension was further cooled down to 10-15 °C, filtered and washed with 800 mL of water. The product was dried under vacuum at 50 °C to obtain 202 g of light-beige crystals of 97.9 a/a-% HPLC purity (77.6% yield).

## Claims

1. Process for preparing a compound of formula (I) 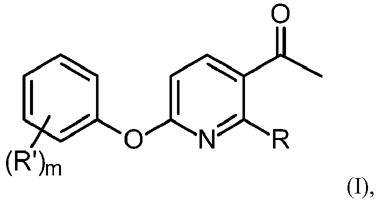 wherein
R represents C₁-C₂-haloalkyl;
R' represents halogen, CN, nitro, C₁-C₄-alkyl, C₁-C₄-haloalkyl, C₁-C₄-alkoxy, C₁-C₄-haloalkoxy, C₁-C₄-alkylcarbonyl, hydroxy-substituted C₁-C₄-alkyl or pentafluoro-λ⁶-sulfanyl; and
m is an integer and is 0, 1, 2, 3, 4 or 5;
**characterized in that** a compound of formula (II) 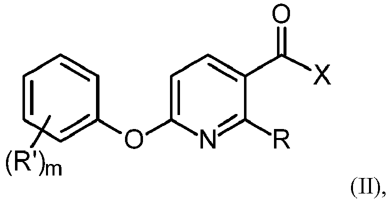 wherein
R, R' and m are defined as in formula (I); and
X represents chlorine or bromine;
is reacted in a first step A1) with a malonate of formula (III) 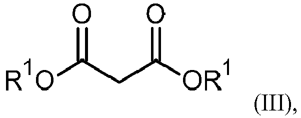 wherein
R¹ represents C₁-C₆-alkyl, C₂-C₆-alkenyl, C₂-C₆-alkynyl, C₃-C₈-cycloalkyl or benzyl; in presence of a base and a magnesium compound; and in a second step A2) the resulting compound is decarboxylated.

2. Process according to claim 1, wherein R represents CF₃.

3. Process according to at least one of claims 1 to 2, wherein R' represents Br or Cl and/or m is 1.

4. Process according to at least one of claims 1 to 3, wherein X represents chlorine.

5. Process according to at least one of claims 1 to 4, wherein R¹ represents methyl, ethyl, n-propyl, isopropyl, n-butyl, isobutyl or tert.-butyl.

6. Process according to at least one of claims 1 to 5, wherein the base present in step A1) is selected from trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, 1,4-diazabicyclo[2.2.2]-octane (DABCO), 1,5-diazabicyclo[4.3.0]-non-5-ene (DBN), 1,8-diazabicyclo[5.4.0]-undec-7-ene (DBU) and mixtures thereof.

7. Process according to at least one of claims 1 to 6, wherein the magnesium compound present in step A1) is selected from MgCl₂, MgBr₂, Mg(OH)₂, Mg(OMe)₂, Mg(OEt)₂, MgO, Mg(NO₃)₂ and mixtures thereof.

8. Process according to at least one of claims 1 to 7, wherein in step A1) the molar ratio of compound of formula (II) and magnesium compound is 1 : 0.4 to 1 : 0.8.

9. Process according to at least one of claims 1 to 8, wherein decarboxylation is performed by heating the compound resulting from step A1) in the presence of water to 50 to 180°C.

10. Process according to at least one of claims 1 to 9, wherein step A1) is conducted in the presence of an organic solvent selected from toluene, xylene, mixtures thereof, and mixtures thereof with acetonitrile or ethylacetate.

11. Process according to at least one of claims 1 to 10, wherein the compound of formula (II) is prepared by reacting a compound of formula (V) 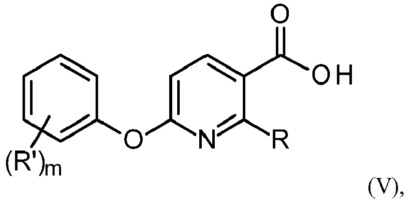 wherein
R, R' and m are defined as in formula (II);
in a step B) with a dehydroxyhalogenation agent selected from COCl₂, diphosgene, triphosgene, cyanuric chloride, SOCl₂, SO₂Cl₂, PCl₃, PCl₅, POCl₃, PBr₃, SOBr₂ and SO₂Br₂.

12. Process according to at least one of claims 1 to 11, wherein compound of formula (I) is further reacted to a triazole derivative of formula (VI) 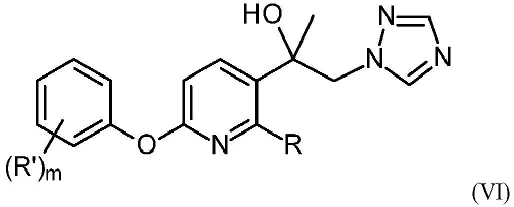 wherein
R, R' and m are defined as in formula (I);
**characterized in that**
the reaction of the compound of formula (I) to the triazole derivative of formula (VI) comprises the following steps :
step C):
reacting the compound of formula (I) with a trimethylsulfoxonium halide, a trimethylsulfonium halide, trimethylsulfoxonium methylsulfate or trimethylsulfonium methylsulfate to an epoxide of formula (VII) 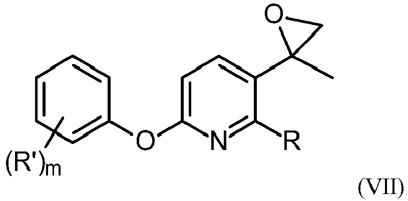 wherein
R, R' and m are defined as in formula (VI); and
step D):
reacting the compound of formula (VII) with 1H-1,2,4-triazole in the presence of a base to the triazole derivative of formula (VI).

13. Compound of formula (IVa) or (IVb) 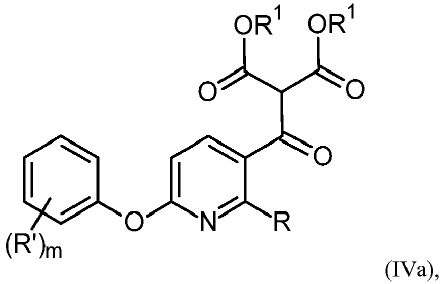 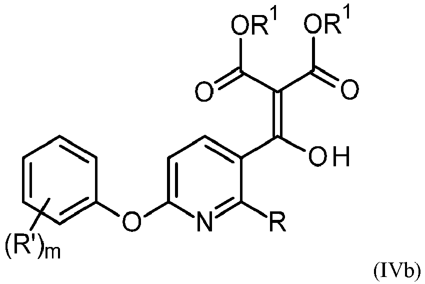 wherein
R represents C₁-C₂-haloalkyl;
R' represents halogen, CN, nitro, C₁-C₄-alkyl, C₁-C₄-haloalkyl, C₁-C₄-alkoxy, C₁-C₄-haloalkoxy, C₁-C₄-alkylcarbonyl, hydroxy-substituted C₁-C₄-alkyl or pentafluoro-λ⁶-sulfanyl;
m is an integer and is 0, 1, 2, 3, 4 or 5; and
R¹ represents C₁-C₆-alkyl, C₂-C₆-alkenyl, C₂-C₆-alkynyl, C₃-C₈-cycloalkyl or benzyl.

14. Compound according to claim 13, wherein
R represents CF₃;
R' represents chlorine or bromine in the 4-position of the phenyl ring;
m is 1; and
R¹ represents CH3.

15. Compound of formula (II) 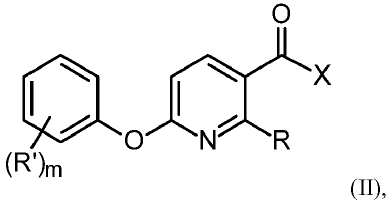 wherein
R represents C₁-C₂-haloalkyl;
R' represents halogen, CN, nitro, C₁-C₄-alkyl, C₁-C₄-haloalkyl, C₁-C₄-alkoxy, C₁-C₄-haloalkoxy, C₁-C₄-alkylcarbonyl, hydroxy-substituted C₁-C₄-alkyl or pentafluoro-λ⁶-sulfanyl;
m is an integer and is 0, 1, 2, 3, 4 or 5; and
X represents chlorine or bromine.

16. Compound according to claim 15, wherein
R represents CF₃;
R' represents chlorine or bromine in the 4-position of the phenyl ring;
m is 1; and
X represents chlorine.
